# EUROPEAN PATENT APPLICATION

(11) **EP 4 159 108 A1**
(43) Date of publication of application: **05.04.2023**
(21) Application number: 21812744.7
(22) Date of filing: 28.05.2021
(51) Int. Cl.: A61B 1/045

(54) **ENDOSCOPE PROCESSOR DEVICE**

(30) Priority: 28.05.2020 JP 2020093057
(71) Applicant: FUJIFILM Corporation, Tokyo 106-8620 (JP)
(72) Inventor: KITAHARA, Masahiro, Tokyo 106-8620 (JP)
(74) Representative: Jeffrey, Philip Michael
(86) International application number: PCT/JP2021/020413
(87) International publication number: WO 2021/241735

(57) **Abstract**

Provided is an endoscope processor device capable of supporting an examination in accordance with a procedure defined by guidelines.

An observation scene information acquiring unit (22) acquires observation scene information regarding an imaged observation scene. An observation scene comparing unit (25) compares reference scene information (31) regarding a reference scene predefined by guidelines with the observation scene information. A recommended observation scene reporting unit (26) displays recommended observation scene information on a display (18) in response to the observation scene not being a recommended observation scene.

## Description

### BACKGROUND OF THE INVENTION

### 1. Field of the Invention

The present invention relates to an endoscope processor device that performs recognition processing using artificial intelligence (AI).

### 2. Description of the Related Art

In the medical field, diagnoses using an endoscope system including an endoscope, a light source device, and a processor device are widely being performed. An endoscope system generates an image of an observation target on the basis of an image signal captured by an endoscope and displays the image on a display. A user (doctor) is able to make a diagnosis while viewing the observation image displayed on the display.

In recent years, AI technology has begun to be introduced into endoscope systems in order to efficiently support diagnosis. Such an endoscope system is particularly useful in a screening examination, which is a method of quickly determining the possibility of a disease. The following patent documents describe a technique of supporting diagnosis.

JP2018-139846A discloses a technique of comparing an examination image with a standard image, promoting an imaging operation if the degree of match therebetween is high, and providing support so that an image conforming to guidelines can be acquired. JP2017-99509A discloses a support technique for performing an examination conforming to an examination protocol by detecting an executed task in accordance with the details of a treatment and displaying the executed task on a display such that the executed task is distinguished from an unexecuted task. JP2012-70936A discloses a technique of generating an alert to notify a user of a failure in capturing images in a case where the number of images of a predetermined examination portion does not match a scheduled number of images to be captured.

### SUMMARY OF THE INVENTION

Guidelines for an endoscopic examination include not only the locations of regions to be observed, but also procedures such as the imaging order of targets to be observed. This aims at preventing insufficient observation or false negative and shortening an examination time by making the region of an organ appearing in an image for secondary interpretation continuous and reducing unnecessary movement of a scope during the examination.

However, in the methods according to JP2018-139846A and JP2012-70936A, the user has to move an endoscope to an appropriate imaging position in order to acquire an image conforming to the guidelines. Considering that a complicated operation is necessary to acquire an appropriate image, these methods are not effective to quickly perform screening. In the method according to JP2017-99509A, support information is displayed so that an examination can be performed according to a predetermined procedure. However, if imaging is insufficiently performed due to the presence of a lesion in an observation target, a state of illumination, or the like, it may be difficult even for a skilled user to perform imaging at an appropriate position unless specific operation information is provided.

It is an object of the present invention to provide an endoscope processor device capable of supporting an endoscopic examination conforming to a procedure defined by guidelines.

An endoscope processor device of the present invention includes a processor configured to acquire a captured medical image; in response to a specific process being performed, acquire observation scene information regarding an imaged observation scene by using the medical image; store the observation scene information in an observation scene information memory; store reference scene information regarding a reference scene predefined by guidelines in a reference scene information memory; compare the reference scene information with the observation scene information and determine whether the observation scene is a recommended observation scene to be observed in accordance with the guidelines; and in response to the observation scene not being the recommended observation scene, display recommended observation scene information regarding the recommended observation scene on a display.

Preferably, the processor is configured to store, in the observation scene information memory, the observation scene and an ordinal number of an imaging order in which the specific process has been performed in association with each other; store, in the reference scene information memory, the reference scene and an ordinal number of an imaging order of the reference scene defined by the guidelines; and compare the observation scene with the reference scene corresponding to the ordinal number of the imaging order of the observation scene and determine whether the observation scene is the recommended observation scene.

Preferably, the processor is configured to, in response to determining that the observation scene is not the recommended observation scene, display, on the display, the reference scene information on the reference scene corresponding to the ordinal number of the imaging order as the recommended observation scene information.

Preferably, the processor is configured to, in response to determining that the observation scene is the recommended observation scene, display, on the display, the reference scene information on a reference scene corresponding to an ordinal number next to the ordinal number of the imaging order as the recommended observation scene information.

Preferably, the specific process is a still image acquisition process of acquiring a still image of the medical image.

Preferably, the processor is configured to display a guide for leading an endoscope to the recommended observation scene.

Preferably, the processor is configured to display the guide in such a manner that the guide is superimposed on the medical image.

Preferably, the guide includes a procedure of an operation for imaging the recommended observation scene on the basis of the reference scene information.

Preferably, the processor is configured to display, as text information, at least information regarding a part including the observation scene and a position of the observation scene of the observation scene information.

Preferably, the processor is configured to, in response to all of the reference scenes being imaged, detect an observation scene to be reexamined among the observation scenes and display information on the observation scene to be reexamined.

According to the present invention, it is possible to support an endoscopic examination conforming to a procedure defined by guidelines.

### BRIEF DESCRIPTION OF THE DRAWINGS

Fig. 1 is an external appearance diagram of an endoscope system;
Fig. 2 is a block diagram illustrating functions of an endoscope processor device;
Fig. 3 is a diagram illustrating a procedure of operation of an endoscope in the case of performing a screening examination on an upper digestive tract;
Fig. 4 is a diagram describing reference scene information;
Fig. 5 is a diagram illustrating an example in which a captured medical image and related information are displayed on a monitor;
Fig. 6 is a diagram describing comparison between observation scenes in an observation scene information memory and reference scenes in reference scene information in a case where imaging is performed in accordance with guidelines;
Fig. 7 is a diagram describing a screen displayed in a case where imaging is performed in accordance with guidelines;
Fig. 8 is a diagram describing comparison between observation scenes in the observation scene information memory and reference scenes in the reference scene information in a case where imaging is not performed in accordance with guidelines;
Fig. 9 is a diagram describing a screen displayed in a case where imaging is not performed in accordance with guidelines;
Fig. 10 is a diagram describing display of a guide for leading an operation of an endoscope to image a recommended observation scene;
Fig. 11 is a diagram describing a first example of displaying a guide by superimposing it on a medical image;
Fig. 12 is a diagram describing a second example of displaying a guide by superimposing it on a medical image;
Fig. 13 is a diagram describing a procedure of operation of an endoscope stored for imaging reference scenes defined by guidelines in accordance with the guidelines;
Fig. 14 is a block diagram illustrating functions of an endoscope processor device including an observation-scene-to-be-reexamined detecting unit; and
Fig. 15 is a flowchart describing functions of a recommended observation scene reporting unit.

### DESCRIPTION OF THE PREFERRED EMBODIMENTS

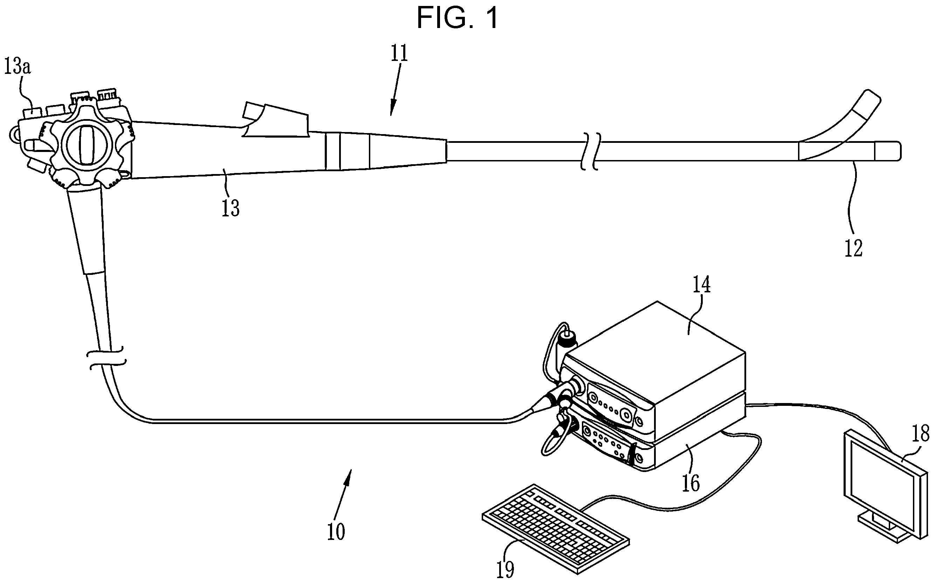

As illustrated in Fig. 1, an endoscope system 10 has an endoscope 11, a light source device 14, an endoscope processor device 16, a display 18, and a user interface 19. The endoscope 11 is optically connected to the light source device 14 and is electrically connected to the endoscope processor device 16. The endoscope 11 illuminates an observation target with illumination light supplied from the light source device 14. In addition, the endoscope 11 images the observation target and acquires an image signal. The endoscope processor device 16 acquires the image signal from the endoscope 11 and performs various types of image processing on the image signal. In addition, the endoscope processor device 16 displays various images on the display 18 on the basis of the image signal subjected to the various types of image processing.

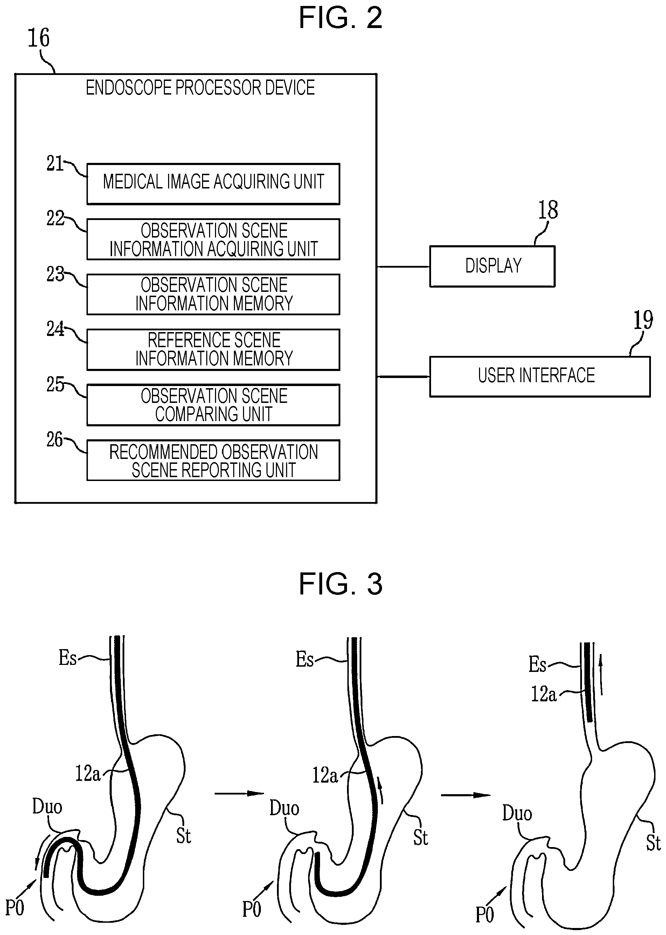

As illustrated in Fig. 2, the endoscope processor device 16 has a medical image acquiring unit 21, an observation scene information acquiring unit 22, an observation scene information memory 23, a reference scene information memory 24, an observation scene comparing unit 25, and a recommended observation scene reporting unit 26. The individual functions of the endoscope processor device 16 are implemented by a control unit (not illustrated) constituted by a processor executing a program for recognizing a lesion or the like incorporated in a program memory.

The endoscope processor device 16 is electrically connected to the display 18 and the user interface 19. The display 18 displays an image of an observation target, information accompanying the image, and so forth. The user interface 19 receives an input operation, such as designation of a region of interest (ROI) or setting of a function.

The endoscope system 10 has a normal-light mode, a special-light mode, and an examination support mode as observation modes. The medical image acquiring unit 21 generates a medical image on the basis of an image signal obtained by imaging an observation target in an environment that varies according to an observation mode.

In a case where the observation mode is the normal-light mode, normal light such as white light is emitted, and a normal-light image having a natural color tone is generated on the basis of an image signal obtained by imaging an observation target illuminated with the normal light. In a case where the observation mode is the special-light mode, special light having an emission spectrum different from that of normal light is emitted, and a special-light image having an emphasized lesion portion and structure is generated on the basis of an image signal obtained by imaging an observation target illuminated with the special light.

In a case where the observation mode is the examination support mode, examination illumination light is emitted. Subsequently, an examination image is generated on the basis of an image signal obtained by imaging an observation target in an examination region illuminated with the examination illumination light. The examination support mode is a mode of providing support for reliably capturing an image defined by medical examination guidelines during screening. Normal light or special light can be used as the examination illumination light.

For example, in the case of performing screening on an upper digestive tract in accordance with the guidelines, as illustrated in Fig. 3, an insertion section 12 of the endoscope 11 is pushed into the lumen, and is then inserted through an esophagus Es and a stomach St to a deepest examination position P0 of a duodenum Duo. Upon the deepest examination position P0 being reached, capturing of a still image is started. First, the user operates an operation section 13 of the endoscope 11 to image an examination region defined by the guidelines at the deepest examination position P0. In the upper digestive tract, examination regions defined by the guidelines are imaged while the insertion section 12 of the endoscope 11 is withdrawn from the duodenum Duo, the stomach St, and the esophagus Es. After all the examination regions have been imaged, the insertion section 12 of the endoscope 11 is withdrawn from the body cavity. It is preferable to perform screening in a similar procedure for a lower digestive tract, such as a large intestine.

Measuring graduations (not illustrated) for measuring an insertion length, which is a length over which the insertion section 12 is inserted into the body, are provided on an outer peripheral surface of the insertion section 12 of the endoscope 11. The measuring graduations are marked at a predetermined pitch (for example, 1 cm pitch) in the longitudinal direction of the insertion section 12. The insertion length is measured by reading the value of the measurement graduations by a graduations detection sensor (not illustrated). The graduations detection sensor is preferably provided on a mouthpiece that a patient holds in the mouth when the insertion section 12 of the endoscope 11 is to be inserted. The graduations detection sensor is connected to the endoscope processor device 16 in a wired or wireless manner, and information detected by the graduations detection sensor is transmitted to the endoscope processor device 16.

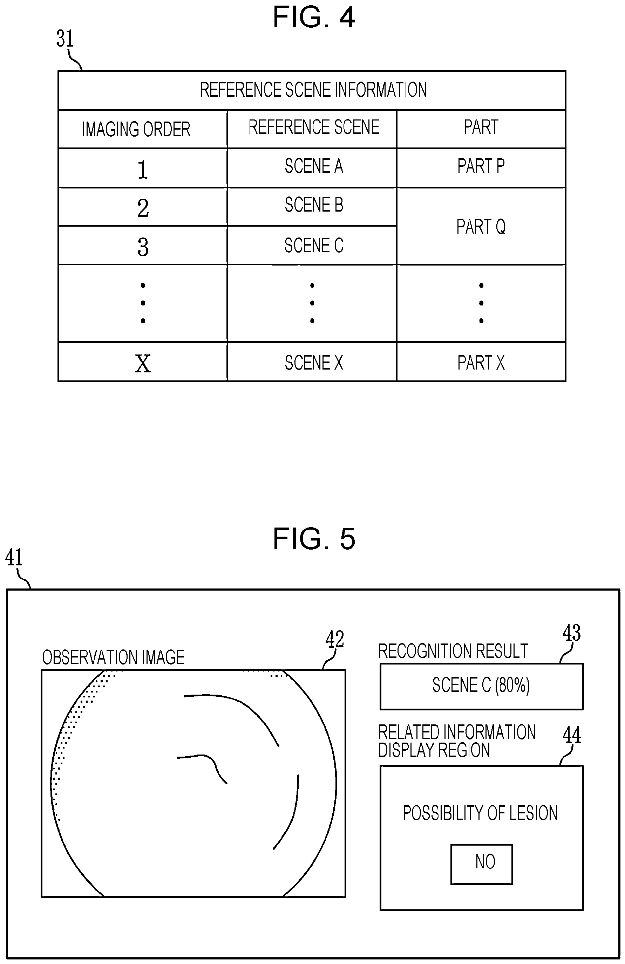

The reference scene information memory 24 stores reference scene information 31. As illustrated in Fig. 4, the reference scene information 31 stores reference scenes and ordinal numbers of an imaging order of the reference scenes defined by the guidelines in association with each other. For example, in the reference scene information 31, the reference scene that is the first in the imaging order is scene A of a small region of part P. Similarly, the second reference scene is scene B of a small region of part Q, and the third reference scene is scene C of a small region of part Q. In this way, the reference scene information 31 stores all the reference scenes defined by the guidelines together with the ordinal numbers of the imaging order.

Fig. 5 illustrates an example of a display screen 41 displayed on the display 18 when a screening examination is performed. On the display 18, an observation image 42 obtained by imaging an observation target is displayed in real time, a scene recognition result 43 of an observation scene that is being observed is displayed, and information regarding the observation scene, such as the possibility of presence or absence of a lesion, is displayed in a related information display region 44. In Fig. 5, the scene recognition result 43 is displayed indicating that the observation scene is "scene C" and that the similarity of the observation scene with scene C is "80%". The similarity is calculated by comparing the observation image 42 with an image in which an observation scene is stored in advance. As the observation image 42, an examination image captured using examination illumination light is displayed during screening, which may be a normal-light image or a special-light image. When a still image is captured by operating the endoscope 11, the captured still image may be displayed.

When a freeze button 13a (see Fig. 1) provided in the operation section 13 of the endoscope 11 has been operated and a still image acquisition process (specific process) of imaging an observation scene displayed on the display 18 as a still image has been performed, the observation scene information acquiring unit 22 acquires observation scene information regarding the imaged observation scene. The observation scene information memory 23 stores the acquired observation scene information in association with an ordinal number of an imaging order indicating the order in which the still image acquisition process has been performed. The observation scene information includes information on a part including an imaging region and the specific position thereof, presence or absence of a lesion in the imaging region, and so forth. These pieces of information are acquired as a result of processing executed by so-called artificial intelligence (AI) using a learning model based on machine learning. Preferably, the similarity of an observation scene with a reference scene, which will be described below, is also processed by AI. A convolutional neural network (CNN) may be used as machine learning.

The observation scene comparing unit 25 compares the observation scene information stored in the observation scene information memory 23 with the reference scene information 31 stored in the reference scene information memory 24, and determines whether the observation scene is a recommended observation scene to be observed in accordance with the guidelines.

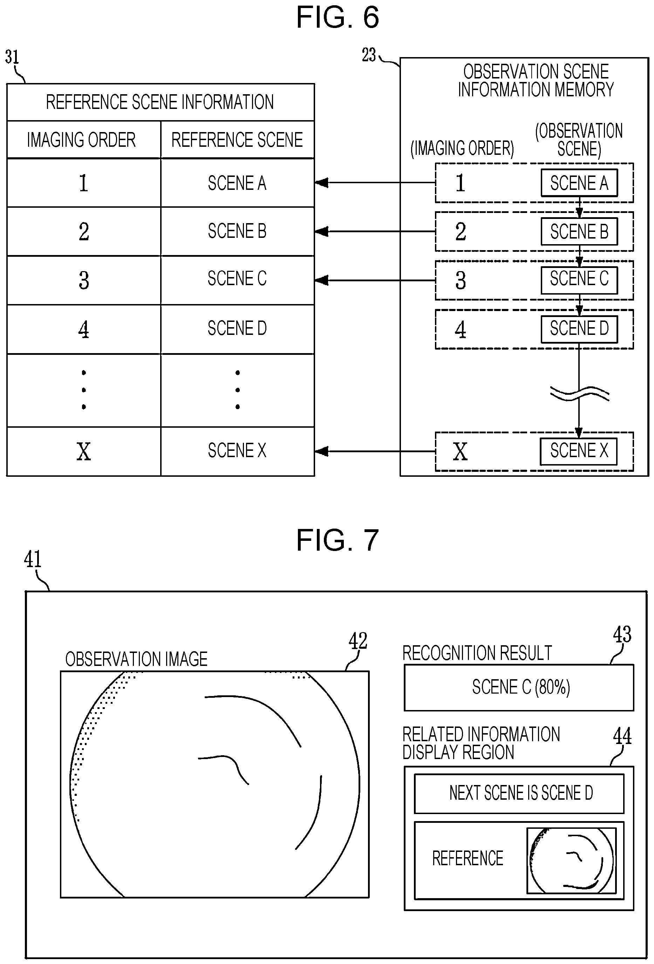

Specifically, the observation scene comparing unit 25 compares the observation scene with the reference scene corresponding to the ordinal number of the imaging order of the observation scene, and determines whether the observation scene is a recommended observation scene. As illustrated in Fig. 6, for example, the observation scene that is the first in the imaging order stored in the observation scene information memory 23 is "scene A". This is compared with "scene A" corresponding to the first in the imaging order of the reference scene information 31. In this way, the observation scene information memory 23 sequentially compares the scenes associated with the same ordinal numbers of the imaging order in accordance with the imaging order.

If the observation scene comparing unit 25 determines that the observation scene is a recommended observation scene, the recommended observation scene reporting unit 26 displays, in the related information display region 44 of the display 18, the reference scene information 31 on the reference scene corresponding to the ordinal number next to the ordinal number of the imaging order of the observation scene.

In Fig. 6, "scene C" is stored as the observation scene that is the third in the imaging order in the observation scene information memory 23. If this scene is the same as the third reference scene in the reference scene information 31, the observation scene is determined to be a recommended observation scene. At this time, as illustrated in Fig. 7, information on the fourth "scene D" is displayed in the related information display region 44 of the display 18 as the reference scene information 31 on the next scene of "scene C".

On the other hand, if the observation scene comparing unit 25 determines that the observation scene is not a recommended observation scene, the recommended observation scene reporting unit 26 displays, in the related information display region 44 of the display 18, the reference scene information 31 on the reference scene corresponding to the ordinal number of the imaging order of the observation scene, as recommended observation scene information.

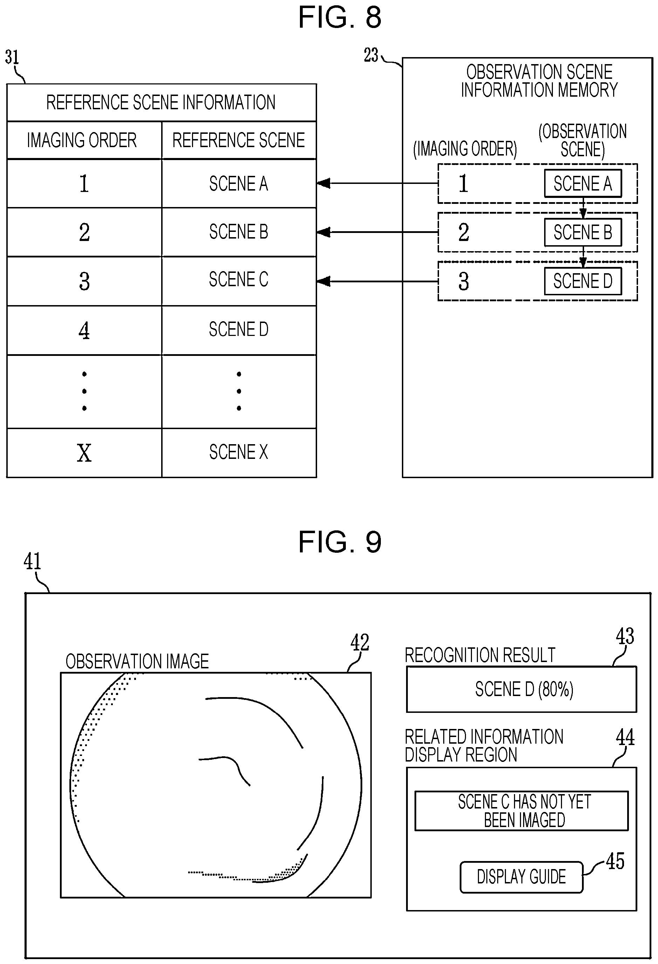

As illustrated in Fig. 8, "scene D" is stored as the observation scene that is the third in the imaging order in the observation scene information memory 23. As compared with the reference scene that is the third in the imaging order in the reference scene information 31, the third reference scene in the reference scene information 31 is "scene C", which is different. At this time, the observation scene comparing unit 25 determines that the observation scene is not a recommended observation scene.

In this case, the recommended observation scene reporting unit 26 displays the reference scene "scene C" that is the third in the imaging order as recommended observation scene information in the related information display region 44. As a method for displaying the recommended observation scene information, for example, as illustrated in Fig. 9, "scene C has not yet been imaged" is displayed in the related information display region 44 in order to report that "scene C" has not yet been imaged as the scene that is the third in the imaging order.

When all the reference scenes defined by the guidelines have been imaged, if the captured images include an image determined to have a high possibility of a lesion, it is preferable to display, as a recommended observation scene to be imaged next, information regarding the observation scene of the image or operation information for moving to the observation scene.

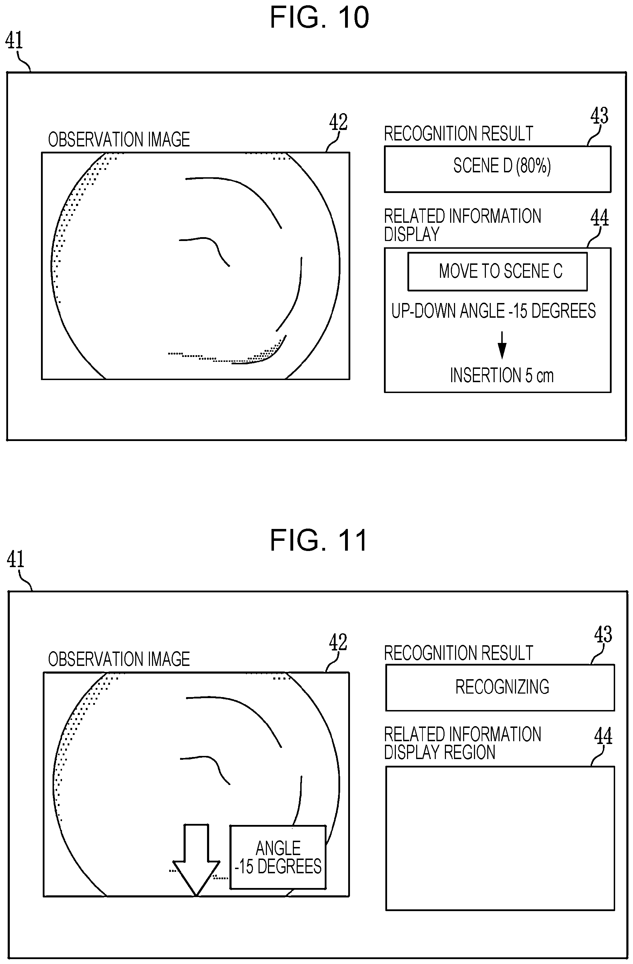

In addition, as illustrated in Fig. 10, the recommended observation scene reporting unit 26 displays a guide for leading a scope (not illustrated) of the endoscope 11 to a position where the recommended observation scene can be imaged. For example, in the related information display region 44 of the display 18, "up-down angle -15 degrees" and "insertion 5 cm" are displayed as operation information for leading to the position where scene C is to be imaged.

The guide may be displayed so as to be superimposed on the observation image 42 displayed on the display 18. In this case, operation information can be presented by, for example, displaying a figure indicating the direction and angle in which the scope of the endoscope 11 is to be moved, or changing the color of the frame portion of the observation image 42 to a color corresponding to operation information so as to indicate the moving direction.

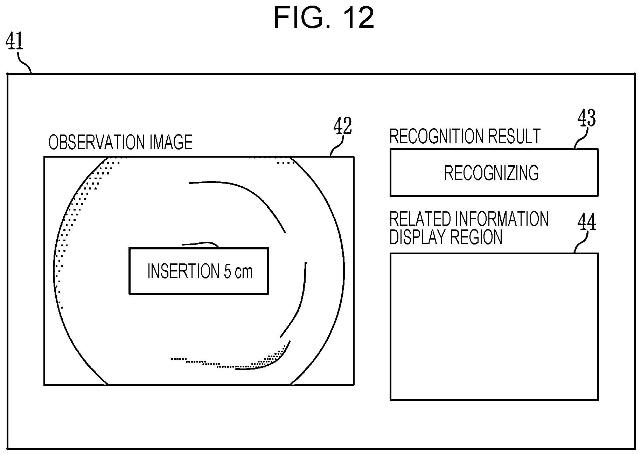

In the case of superimposing a guide on the observation image 42, for example, a downward arrow and "-15 degrees" are displayed to indicate that the angle of the scope of the endoscope 11 is to be operated by 15 degrees in the negative direction (downward direction), as illustrated in Fig. 11. Furthermore, as illustrated in Fig. 12, "insertion 5 cm" is displayed to indicate an operation of inserting the scope of the endoscope 11 by 5 cm. In this way, in the case of displaying the guide by superimposing it on the observation image 42, it is preferable to switch the display for each operation.

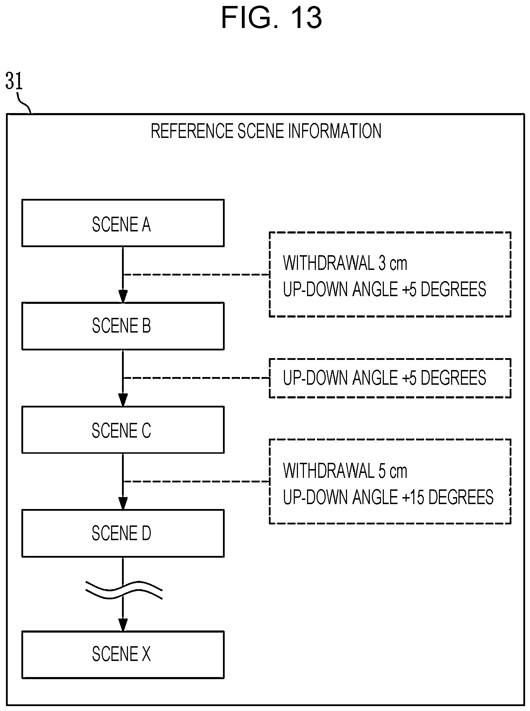

As illustrated in Fig. 13, the reference scene information 31 stores operation procedures of moving the scope of the endoscope 11 from the position where the preceding reference scene is imaged to the position where the next reference scene is imaged. These operation procedures are stored in advance in association with information regarding physiques such as heights. The above-described guide is displayed in accordance with these operation procedures. For example, in the case of moving the scope of the endoscope 11 to return from scene D to scene C, the guide is displayed to indicate a procedure reverse to the operation procedure from scene C to scene D. That is, the guide is displayed so that the up-down angle is changed by -15 degrees and then an insertion operation of 5 cm is performed.

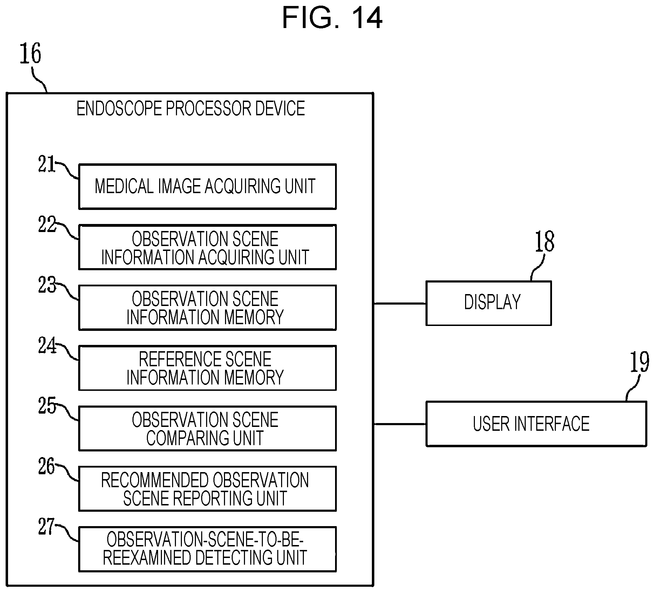

As illustrated in Fig. 14, the endoscope processor device 16 may include an observation-scene-to-be-reexamined detecting unit 27. The observation-scene-to-be-reexamined detecting unit 27 detects, after all the reference scenes have been imaged, an observation scene that needs to be reexamined among the observation scenes stored in the observation scene information memory 23. For example, in response to detecting a suspicious lesion region in scene B among the stored observation scenes (see Fig. 6 and so forth), the observation-scene-to-be-reexamined detecting unit 27 detects scene B as an observation scene to be reexamined. In this case, the recommended observation scene reporting unit 26 displays information regarding scene B and a guide for operating the endoscope 11 on the display 18.

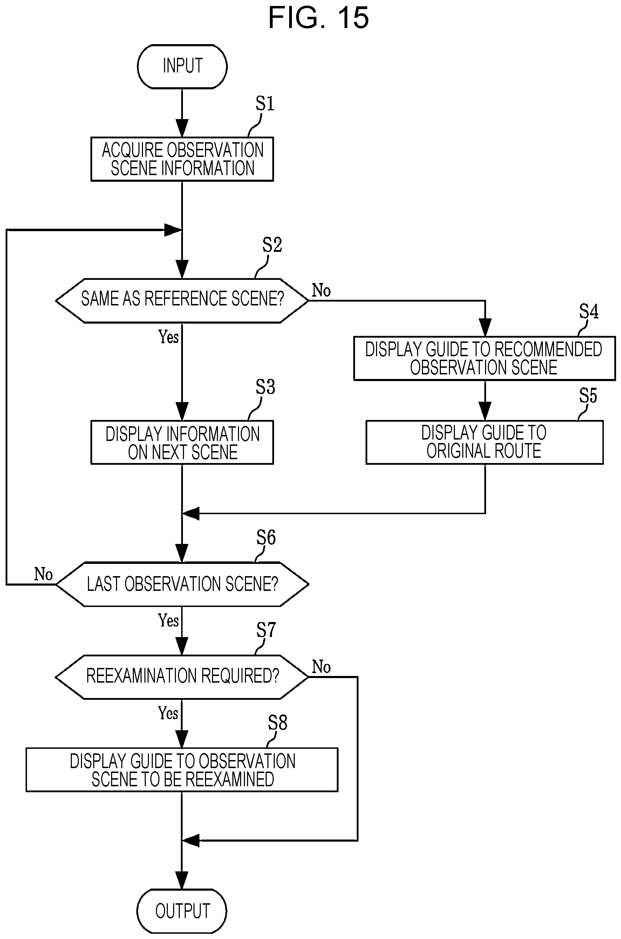

Fig. 15 is a flowchart illustrating the functions of the recommended observation scene reporting unit 26 when the user performs a screening examination by using the endoscope system 10 of the present invention. First, in response to an observation scene being imaged as a still image by the user, the observation scene information acquiring unit 22 stores observation scene information, in which the observation scene is associated with an ordinal number of an imaging order, in the observation scene information memory 23 (step S1).

The observation scene comparing unit 25 compares the observation scene stored in the observation scene information memory 23 with the reference scene stored in the reference scene information 31, and determines whether the observation scene is a recommended observation scene to be observed in accordance with the guidelines (step S2). If it is determined that the stored observation scene is a recommended observation scene, the recommended observation scene reporting unit 26 displays, on the display 18, the reference scene information 31 on the reference scene corresponding to the ordinal number next to the ordinal number of the imaging order of the observation scene, as recommended observation scene information (step S3). If it is determined that the stored observation scene is not a recommended observation scene, it is determined that imaging has not been performed in accordance with the guidelines. In this case, the reference scene information 31 on the reference scene corresponding to the ordinal number of the imaging order of the observation scene is displayed as recommended observation scene information on the display 18 (step S4). In step S4, a guide for leading the scope of the endoscope 11 to a position where the recommended observation scene can be imaged is further displayed on the display 18, and the user performs imaging on a not yet imaged observation scene in accordance with the guide. After imaging of the recommended observation scene has finished, the recommended observation scene reporting unit 26 displays a guide for returning to the original route (step S5).

In a similar procedure, the user performs the screening examination to reach the last observation scene in accordance with the guidelines. If the last imaged observation scene is the last scene of the reference scene information 31, the observation scene comparing unit 25 determines that the observation scene is the last observation scene (step S6). The observation-scene-to-be-reexamined detecting unit 27 detects, after all the reference scenes have been imaged, an observation scene that needs to be reexamined among the observation scenes stored in the observation scene information memory 23 (step S7). The recommended observation scene reporting unit 26 displays information regarding the observation scene to be reexamined and a guide for operating the endoscope 11 on the display 18 (step S8). After finishing all examinations including the reexamination, the user withdraws the endoscope 11 to the outside of the body cavity and finishes the examinations.

In the present embodiment, whether imaging has been performed in accordance with the guidelines is determined at the timing when the user has imaged an observation scene as a still image. Also for an image continuously captured, such as a moving image, whether the guidelines are fulfilled can be determined by using the following method. For example, the observation scene comparing unit 25 determines whether a reference scene in the reference scene information 31 is included in an image captured as a moving image. If the examination is performed in accordance with the guidelines, specific scenes (reference scenes) are included in the moving image in the same order as the predetermined imaging order. Thus, if the order of the observation scenes imaged as a moving image is different from the order of the reference scenes, the observation scene comparing unit 25 determines that the guidelines are not fulfilled. If it is determined that the guidelines are not fulfilled, the recommended observation scene reporting unit 26 displays operation information to guide the user so that the user can perform imaging in accordance with the guidelines.

In the present embodiment, the hardware structure of a processing unit that executes various processing operations, such as the medical image acquiring unit 21, the observation scene information acquiring unit 22, the observation scene comparing unit 25, and the recommended observation scene reporting unit 26, may be various types of processors described below. The various types of processors include a central processing unit (CPU), which is a general-purpose processor executing software (program) and functioning as various processing units; a programmable logic device (PLD), which is a processor whose circuit configuration is changeable after manufacturing, such as a field programmable gate array (FPGA); a dedicated electric circuit, which is a processor having a circuit configuration designed exclusively for executing various processing operations, and the like.

A single processing unit may be constituted by one of these various types of processors or may be constituted by a combination of two or more processors of the same type or different types (for example, a combination of a plurality of FPGAs or a combination of a CPU and an FPGA). A plurality of processing units may be constituted by a single processor. Examples of constituting a plurality of processing units by a single processor are as follows. First, as represented by a computer of a client or server, a single processor is constituted by a combination of one or more CPUs and software, and the processor functions as a plurality of processing units. Secondly, as represented by a system on chip (SoC), a processor in which a single integrated circuit (IC) chip implements the function of an entire system including a plurality of processing units is used. In this way, various types of processing units are constituted by using one or more of the above-described various types of processors as a hardware structure.

Furthermore, the hardware structure of the various types of processors is, more specifically, electric circuitry formed by combining circuit elements such as semiconductor elements.

### Reference Signs List

- 10: endoscope system
- 11: endoscope
- 12: insertion section
- 13: operation section
- 13a: freeze button
- 14: light source device
- 16: endoscope processor device
- 18: display
- 19: user interface
- 21: medical image acquiring unit
- 22: observation scene information acquiring unit
- 23: observation scene information memory
- 24: reference scene information memory
- 25: observation scene comparing unit
- 26: recommended observation scene reporting unit
- 27: observation-scene-to-be-reexamined detecting unit
- 31: reference scene information
- 41: display screen
- 42: observation image
- 43: scene recognition result
- 44: related information display region
- 45: guide display button

## Claims

1. An endoscope processor device comprising:
a processor configured to
acquire a captured medical image,
in response to a specific process being performed, acquire observation scene information regarding an imaged observation scene by using the medical image,
store the observation scene information in an observation scene information memory,
store reference scene information regarding a reference scene predefined by guidelines in a reference scene information memory,
compare the reference scene information with the observation scene information and determine whether the observation scene is a recommended observation scene to be observed in accordance with the guidelines, and
in response to the observation scene not being the recommended observation scene, display recommended observation scene information regarding the recommended observation scene on a display.

2. The endoscope processor device according to claim 1, wherein
the processor is configured to
store, in the observation scene information memory, the observation scene and an ordinal number of an imaging order in which the specific process has been performed in association with each other,
store, in the reference scene information memory, the reference scene and an ordinal number of an imaging order of the reference scene defined by the guidelines, and
compare the observation scene with the reference scene corresponding to the ordinal number of the imaging order of the observation scene and determine whether the observation scene is the recommended observation scene.

3. The endoscope processor device according to claim 2, wherein the processor is configured to, in response to determining that the observation scene is not the recommended observation scene, display, on the display, the reference scene information on the reference scene corresponding to the ordinal number of the imaging order as the recommended observation scene information.

4. The endoscope processor device according to claim 2, wherein the processor is configured to, in response to determining that the observation scene is the recommended observation scene, display, on the display, the reference scene information on a reference scene corresponding to an ordinal number next to the ordinal number of the imaging order as the recommended observation scene information.

5. The endoscope processor device according to any one of claims 1 to 4, wherein the specific process is a still image acquisition process of acquiring a still image of the medical image.

6. The endoscope processor device according to any one of claims 1 to 5, wherein the processor is configured to display a guide for leading an endoscope to the recommended observation scene.

7. The endoscope processor device according to claim 6, wherein the processor is configured to display the guide in such a manner that the guide is superimposed on the medical image.

8. The endoscope processor device according to claim 6 or 7, wherein the guide includes a procedure of an operation for imaging the recommended observation scene on the basis of the reference scene information.

9. The endoscope processor device according to any one of claims 1 to 8, wherein the processor is configured to display, as text information, at least information regarding a part including the observation scene and a position of the observation scene of the observation scene information.

10. The endoscope processor device according to any one of claims 1 to 9, wherein the processor is configured to, in response to all of the reference scenes being imaged, detect an observation scene to be reexamined among the observation scenes and display information on the observation scene to be reexamined.
